(19) Europäisches Patentamt
European Patent Office
Office européen des brevets

(11) Veröffentlichungsnummer: **0 046 556**
**B1**

(12) # EUROPÄISCHE PATENTSCHRIFT

(45) Veröffentlichungstag der Patentschrift: **09.03.83**

(51) Int. Cl.³: **C 07 C119/048**, C 07 C118/02, C 08 G 18/76

(21) Anmeldenummer: **81106366.8**

(22) Anmeldetag: **17.08.81**

(54) **Neue Diisocyanate bzw. Diisocyanatgemische der Diphenylmethanreihe, Verfahren zu ihrer Herstellung, sowie ihre Verwendung als Aufbaukomponente bei der Herstellung von Polyurethankunststoffen nach dem Isocyanat-Polyadditionsverfahren.**

(30) Priorität: **26.08.80 DE 3032128**

(43) Veröffentlichungstag der Anmeldung: **03.03.82 Patentblatt 82/09**

(45) Bekanntmachung des Hinweises auf die Patenterteilung: **09.03.83 Patentblatt 83/10**

(84) Benannte Vertragsstaaten: **BE DE FR GB IT**

(56) Entgegenhaltungen:
**EP A 0 024 323**
**EP A 0 024 665**
**DE A 1 901 993**
**GB A 1 013 710**
**GB A 1 117 066**
**GB A 1 163 264**
**US A 3 152 162**
**US A 3 644 457**

(73) Patentinhaber: **BAYER AG**
**Zentralbereich Patente, Marken und Lizenzen**
**D-5090 Leverkusen 1, Bayerwerk (DE)**

(72) Erfinder: **Knöfel, Hartmut, Dr.**
**Dülmener Weg 21**
**D-5068 Odenthal-Erberich (DE)**
Erfinder: **Brockelt, Michael**
**Neuhauser Weg 1**
**D-5060 Bergisch Gladbach 2 (DE)**

**EP 0 046 556 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Neue Diisocyanate bzw. Diisocyanatgemische der Diphenylmethanreihe, Verfahren zu ihrer Herstellung, sowie ihre Verwendung als Aufbaukomponente bei der Herstellung von Polyurethankunststoffen nach dem Isocyanat-Polyadditionsverfahren

Die Erfindung betrifft neue, $C_2$-$C_{12}$-Alkyl-substituierte Diisocyanate der Diphenylmethanreihe, die insbesondere durch Isocyanatgruppen in 3,4'- und gegebenenfalls in 3,2'-Stellung gekennzeichnet sind, mehrere, voneinander unabhängige Verfahren zu ihrer Herstellung, sowie ihre Verwendung als Isocyanatkomponente bei der Herstellung von Polyurethankunststoffen nach dem Isocyanat-Polyadditionsverfahren.

Unter den technisch und wirtschaftlich als Ausgangsmaterial für Polyurethankunststoffe wichtigen organischen Polyisocyanaten sind insbesondere 2,4-Diisocyanatotoluol bzw. dessen Gemische mit 2,6-Diisocyanatotoluol (TDI) und 4,4'-Diisocyanatodiphenylmethan bzw. dessen Gemische mit 2,2'- oder 2,4'-Diisocyanatodiphenylmethan und/oder mit höherfunktionellen Homologen (MDI) von hervorragender Bedeutung. Obwohl diese aromatischen Polyisocyanate in großtechnischen Mengen weltweit zur Herstellung von Polyurethankunststoffen, insbesondere von Schaumstoffen und Elastomeren Verwendung finden, sind sie mit bestimmten Nachteilen behaftet. So weist beispielsweise TDI einen im Vergleich zu MDI erhöhten Dampfdruck auf, was bei der Verarbeitung dieses Rohstoffs aus physiologischen Gründen die strikte Beachtung entsprechender Vorsichtsmaßnahmen erfordert. Andererseits ist MDI, d. h. insbesondere das auch in Polyisocyanatgemischen der Diphenylmethanreihe meistens als Hauptkomponente vorliegende 4,4'-Diisocyanatodiphenylmethan bei Raumtemperatur ein Festkörper mit einer hohen Neigung zur Kristallisation, so daß diese Rohstoffe oft vor ihrer Verarbeitung entweder durch Erwärmen auf eine Temperatur oberhalb des Schmelzpunkts von 4,4'-Diisocyanatodiphenylmethan oder durch eine chemische Modifizierung beispielsweise eine partielle Urethanisierung (vgl. z. B. US-PS 3 644 457) oder eine partielle Carbodiimidisierung (vgl. z. B. US-PS 3 152 162) verflüssigt werden müssen.

Durch die vorliegende Erfindung werden nunmehr neue aromatische Polyisocyanate zur Verfügung gestellt, die die Vorteile, nicht jedoch die Nachteile von TDI und MDI in sich vereinen. Darüber hinaus weisen die neuen Diisocyanate bzw. Diisocyanatgemische im Unterschied zu 4,4'-Diisocyanatodiphenylmethan Isocyanatgruppen unterschiedlicher Reaktivität auf, was oft bei der Herstellung von Polyurethankunststoffen von Vorteil ist. Außerdem gestattet der Einsatz der neuen erfindungsgemäßen Diisocyanate bzw. Diisocyanatgemische bei der Herstellung von Polyurethankunststoffen den Einbau neuartiger, dem Kohlenwasserstoffgerüst der Diisocyanate entsprechender Hartsegmente in die Polyurethankunststoffe, was eine interessante neue Variationsmöglichkeit bei der Herstellung von Polyurethankunststoffen eröffnet.

Gegenstand der vorliegenden Erfindung sind gegebenenfalls im Gemisch mit bis zu 40 Gew.-%, bezogen auf Gesamtgemisch, an Diisocyanaten der Formel

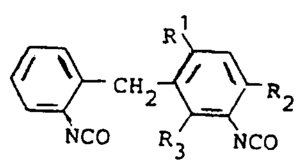

und gegebenenfalls mit bis zu 40 Gew.-%, bezogen auf Gesamtgemisch, an anderen $C_2$-$C_{12}$-Alkyl-substituierten Diisocyanatodiphenylmethan-Isomeren vorliegende Diisocyanate der Formel

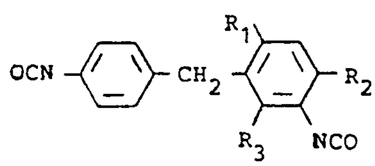

wobei in diesen Formeln die Reste $R_1$, $R_2$ und $R_3$ jeweils gleich oder verschieden sind und Wasserstoff oder eine $C_2$-$C_{12}$-Alkylgruppe bedeuten, mit der Einschränkung, daß in jeder der beiden Formeln jeweils 2 der Reste $R_1$, $R_2$ und $R_3$ für Wasserstoff stehen, und wobei einer der Reste $R_1$, $R_2$ oder $R_3$ für eine Alkylgruppe mit 2-12 C-Atomen, vorzugsweise 2-3 C-Atomen und besonders bevorzugt für eine Ethylgruppe, steht.

Die hier vorgenommene Definition für $R_1$, $R_2$ und $R_3$ gilt auch nachstehend.

Gegenstand der vorliegenden Erfindung ist auch das in diesen Diisocyanaten bzw. Diisocyanatgemischen mit Ethylsubstituenten meistens als Hauptkomponente vorliegende und auch in reiner Form darstellbare Diisocyanat der Formel

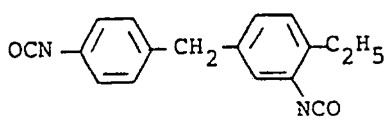

sowie das ebenfalls erfindungsgemäße Diisocyanat der Formel

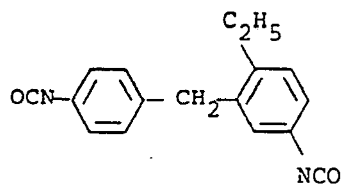

Gegenstand der vorliegenden Erfindung sind auch die nachstehend näher beschriebenen Verfahren zur Herstellung der neuen Diisocyanate bzw. Diisocyanatgemische, sowie die Verwendung der neuen Diisocyante bzw. Diisocyanatgemische als Isocyanatkomponente bei der Herstellung von Polyurethankunststoffen nach dem Isocyanat-Polyadditionsverfahren.

Wie dargelegt, handelt es sich bei den erfindungsgemäßen Verbindungen, die gegebenenfalls als Gemische vorliegen, um Diisocyanate der Diphenylmethanreihe der angegebenen Struktur, welche einen gesättigten Alkylsubstituenten mit 2 bis 12, vorzugsweise 2 oder 3 und insbesondere 2 Kohlenstoffatomen aufweisen. Demzufolge steht im folgenden bei der Beschreibung der erfindungsgemäßen Verfahrensprodukte und auch bei der Beschreibung der bei den erfindungsgemäßen Verfahren eingesetzten Ausgangsmaterialien der Begriff « Alkyl- » stets für einen Substituenten der genannten Art. Dies gilt insbesondere für die gegebenenfalls Nitro-substituierten Alkylbenzole, die bei den erfindungsgemäßen Verfahren als Ausgangsmaterialien eingesetzt werden können.

Die oben und nachstehend gemachten Ausführungen bezüglich der Zusammensetzungen der erfindungsgemäßen Gemische, sowie der Ausgangsmaterialien und Zwischenprodukte beziehen sich auf gaschromatographisch ermittelbare Werte.

Das 1. erfindungsgemäße Verfahren zur Herstellung von erfindungsgemäßen Diisocyanaten ist dadurch gekennzeichnet, daß man

a) ein 4-Nitrobenzylhalogenid in Gegenwart von Friedel-Crafts-Katalysatoren mit 1-Alkyl-2-nitrobenzol und/oder 1-Alkyl-4-nitro-benzol und/oder technischen Gemischen dieser Isomeren mit bis zu 15 Gew.-%, bezogen auf Gesamtgemisch, an 1-Alkyl-3-nitro-benzol zu Dinitroverbindungen der Formel

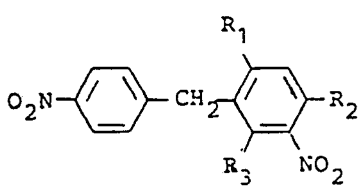

gegebenenfalls im Gemisch mit bis zu 20 Gew.-% an anderen isomeren Dinitro-diphenylmethanen umsetzt, das Umsetzungsprodukt von eingesetztem Katalysator befreit,

b) das gemäß a) erhaltene Umsetzungsprodukt durch Hydrierung oder Reduktion der Nitrogruppen in die entsprechenden aromatischen Diaminoverbindungen überführt und

c) die gemäß b) erhaltenen Diaminoverbindungen durch Phosgenierung in die Diisocyanate überführt,

wobei man gegebenenfalls

d) aus den gemäß b) erhaltenen Diaminen vor der Phosgenierungsreaktion und/oder aus den gemäß c) erhaltenen Diisocyanaten die entsprechenden 3,4'-Diamino- bzw. 3,4'-Diisocyanato-Isomeren destillativ in von Nebenprodukten befreiter Form isoliert.

Unter « Nitrobenzylhalogenid » bzw. « Benzylhalogenid » sind hier und nachstehend vor allem die entsprechenden Benzylchloride bzw. -bromide, insbesondere die entsprechenden Benzylchloride zu verstehen.

Geeignete Nitro- und Alkyl-substituierte Benzole sind hierbei beispielsweise die entsprechenden Mononitrierungsprodukte von Ethylbenzol, Isopropylbenzol, n-Propylbenzol, n-Butyl-benzol, n-Octylbenzol oder n-Dodecyl-benzol.

Bei der Stufe a) des 1. erfindungsgemäßen Verfahrens erfolgt eine Friedel-Crafts-Kondensation zwischen den genannten Nitro-substituierten Alkylbenzolen und 4-Nitrobenzylhalogenid, wobei die

Reaktionspartner in solchen Mengen zum Einsatz gelangen, daß für jedes Mol Nitrobenzylhalogenid 1,0 bis 20, vorzugsweise 2 bis 10 Mol Nitroalkylbenzol zur Verfügung stehen. Die im Überschuß eingesetzte Komponente dient dabei gleichzeitig als Lösungsmittel. Als Katalysatoren dienen die üblichen Friedel-Crafts-Katalysatoren, d. h. z. B. Aluminiumchlorid, Eisentrichlorid, Titantetrachlorid oder Zinntetrachlorid. Vorzugsweise wird Eisentrichlorid als Katalysator verwendet. Die Katalysatoren kommen im allgemeinen in Mengen von 1 bis 100, vorzugsweise 5 bis 50 Mol-%, bezogen auf die Benzylhalogenidkomponente zum Einsatz. Die Umsetzung wird im allgemeinen bei einer zwischen Raumtemperatur und dem Siedepunkt des Reaktionsgemischs, d. h. bei ca. + 20 bis ca. 200 °C, vorzugsweise bei 30 bis 120 °C liegenden Temperatur durchgeführt. Im Anschluß an die Kondensationsreaktion wird der Katalysator vorzugsweise durch Auswaschen z. B. mit Wasser und gegebenenfalls verdünnter Salzsäure entfernt und das überschüssige, nicht umgesetzte Ausgangsmaterial abdestilliert.

Anschließend erfolgt gemäß der Reaktionsstufe b) die Reduktion der Nitrogruppen in die entsprechenden aromatisch gebundenen Aminogruppen. Vorzugsweise erfolgt diese Reduktion durch katalytische Hydrierung beispielsweise unter Verwendung von Raney-Nickel, Platin oder Palladium als Katalysator. Die Hydrierung erfolgt im allgemeinen in alkoholischer Lösung, wobei als Lösungsmittel beispielsweise Methanol, Ethanol, Isopropanol, Toluol oder Gemische daraus zum Einsatz gelangen. Die zu hydrierenden Nitroverbindungen werden hierbei im allgemeinen in Form einer 10-50 gew.-%igen Lösung eingesetzt. Die Hydrierung erfolgt, gegebenenfalls unter Druck, bei einer Temperatur von 20 bis 150 °C, vorzugsweise 30 bis 100 °C. Die Überführung der Nitrogruppen in die entsprechenden Aminogruppen kann selbstverständlich auch nach der an sich bekannten Reduktionsmethode unter Verwendung von beispielsweise Eisen, Zink oder Zinn als Reduktionsmittel erfolgen. Im Anschluß an die Überführung der Nitrogruppen in die entsprechenden Aminogruppen wird der Katalysator beispielsweise durch Filtration entfernt und das Lösungsmittel abdestilliert. Das als Rückstand anfallende Amin kann dann ohne weitere Aufarbeitung dem Reaktionsschritt c) zugeführt werden. Falls auf die Herstellung besonders reiner Verfahrensprodukte Wert gelegt wird, kann aus dem gemäß Reaktionsschritt b) anfallenden Amingemisch das den erfindungsgemäßen Verfahrensprodukten konstitutionell entsprechende Diamin bzw. Diamingemisch auch durch Destillation in von Nebenprodukten befreiter Form hergestellt werden, bevor es dem Reaktionsschritt c) zugeführt wird. Unter « Nebenprodukten » sind hier und auch nachstehend nicht identifizierte niedriger und/oder höher als die erfindungsgemäßen Produkte bzw. Zwischenprodukte siedenden Bestandteile zu verstehen.

Die gegebenenfalls destillativ aufgearbeiteten Diamine werden anschließend gemäß Reaktionsschritt c) in an sich bekannter Weise durch Phosgenierung in die entsprechenden Diisocyanate überführt. Als Lösungsmittel dient hierbei beispielsweise Chlorbenzol oder Dichlorbenzol. Die erfindungsgemäßen Verfahrensprodukte liegen schließlich nach Abdestillieren des Hilfslösungsmittels als Rückstand vor und können gewünschtenfalls durch Destillation in von gegebenenfalls noch vorliegenden Nebenprodukten befreiter Form erhalten werden.

Das 1. erfindungsgemäße Verfahren gestattet bei der Verwendung von 1-Alkyl-2-nitrobenzol als Ausgangsmaterial die Herstellung von Isomerengemischen, die ca. 20 bis 50 Gew.-% 3,4'-Diisocyanato-2-alkyl-diphenylmethan und ca. 50 bis 80 Gew.-% 3,4'-Diisocyanato-4-alkyl-diphenylmethan enthalten und im Falle der Verwendung von 1-Alkyl-4-nitro-benzol als Ausgangsmaterial zur Herstellung von 3,4'-Diisocyanato-6-alkyl-diphenylmethan. Eine Reindarstellung der 3,4'-Diisocyanato-4-alkyl-diphenylmethane ist nach dem Prinzip des 1. erfindungsgemäßen Verfahrens beispielsweise durch Reindarstellung der 3,4'-Dinitro-4-alkyl-diphenylmethane durch partielle Kristallisation aus dem gemäß a) anfallenden Zwischenprodukt und ihre weitere Umsetzung gemäß b) und c) möglich. Diese Reindarstellung durch Kristallisation auf der Nitrostufe erfolgt beispielsweise dergestalt, daß man das nach Abdestillieren von überschüssigem Ausgangsmaterial erhaltene Gemisch von Nitroverbindungen auf Basis von 4-Nitrobenzylhalogenid und o-Nitroalkylbenzolen in siedendem Alkohol oder Essigsäureethylester zu einer gesättigten Lösung löst und die Lösung auf Raumtemperatur abkühlen läßt. Hierbei kristallisiert bevorzugt das gewünschte Isomere aus. Der Kristallisationsvorgang kann selbstverständlich beliebig oft wiederholt werden.

Das 2. erfindungsgemäße Verfahren zur Herstellung erfindungsgemäßer Diisocyanate bzw. Diisocyanatgemische ist dadurch gekennzeichnet, daß man

a) ein 4-Nitrobenzylhalogenid in Gegenwart von Friedel-Crafts-Katalysatoren mit Alkylbenzolen zu einem Gemisch von Mononitroverbindungen der Formel

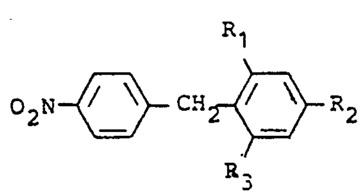

umsetzt, das Umsetzungsprodukt vom Katalysator befreit,

b) das gemäß a) erhaltene Umsetzungsprodukt einer Nitrierungsreaktion unter Bildung von Dinitroverbindungen der Formel

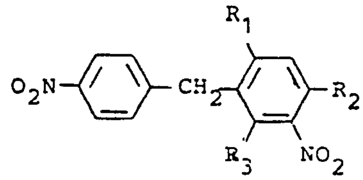

unterzieht,

c) die gemäß b) erhaltenen Dinitroverbindungen durch Hydrierung bzw. Reduktion der Aminogruppen in die entsprechenden aromatischen Diaminoverbindungen überführt und

d) die gemäß c) erhaltenen Diaminoverbindungen durch Phosgenierung in die entsprechenden Diisocyanate überführt,

wobei man gegebenenfalls

e) aus den gemäß c) erhaltenen Diaminen vor der Phosgenierungsreaktion und/oder aus den gemäß d) erhaltenen Diisocyanaten die entsprechenden 3,4'-Diamino- bzw. 3,4'-Diisocyanato-Isomeren destillativ in von Nebenprodukten befreiter Form isoliert.

Die Stufe a) des 2. erfindungsgemäßen Verfahrens entspricht mit der Ausnahme der Verwendung von Alkylbenzolen als Ausgangsmaterial der Stufe a) des 1. erfindungsgemäßen Verfahrens, wobei jedoch im allgemeinen die Siedetemperatur des hier in einem entsprechenden Überschuß eingesetzten Alkylbenzols die obere Grenze des Temperaturbereichs darstellt. Das gemäß a) erhaltene, vom Katalysator befreite Kondensationsprodukt wird anschließend in der Stufe b) in an sich bekannter Weise zu einem Gemisch isomerer Dinitroverbindungen der genannten allgemeinen Formel nitriert. Diese Nitrierung erfolgt beispielsweise in Gegenwart eines geeigneten Lösungsmittels wie Methylenchlorid unter Verwendung von « Nitriersäure », d. h. ein Gemisch aus konzentrierter Schwefelsäure und Salpetersäure, vorzugsweise hochkonzentrierter, ca. 98 %iger Salpetersäure. Die Menge der Nitriersäure wird so bemessen, daß für jedes Mol an gemäß a) erhaltener Mononitroverbindung ca. 1,1 Mol Salpetersäure zur Verfügung stehen. Die Nitrierung wird im allgemeinen im Temperaturbereich zwischen − 20 und 80 °C, vorzugsweise 0-20 °C, durchgeführt. Anschließend wird die nach der Nitrierungsreaktion vorliegende organische Phase von der Säure durch Phasentrennung, Auswaschen mit Wasser und beispielsweise Natriumcarbonatlösung befreit. Schließlich erfolgt die destillative Entfernung des Hilfslösungsmittels gegebenenfalls unter anschließendem Austreiben von Lösungsmittelresten mittels Wasserdampf.

Die weitere Umsetzung der so erhaltenen Dinitroverbindungen erfolgt in völliger Analogie zu den Reaktionsschritten b) und c) des 1. erfindungsgemäßen Verfahrens.

Beim 2. erfindungsgemäßen Verfahren entstehen als erfindungsgemäße Diisocyanatgemische im allgemeinen Gemische, die ca. 70 bis 90 Gew.-% 3,4'-Diisocyanato-2-, -4- oder -6-alkyl-diphenylmethan und 10 bis 40 Gew.-% an anderen, analytisch nicht charakterisierten Alkyl-substituierten Diisocyanatodiphenylmethan-Isomeren enthalten. Die Diisocyanate entsprechen somit in erster Näherung der Formel

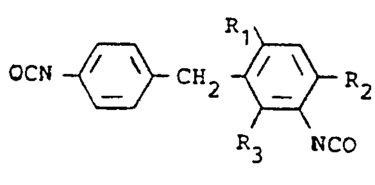

wobei — dies gilt auch für die beiden oben zuletzt genannten Formeln — einer der Reste $R_1$, $R_2$ oder $R_3$ für eine Alkylgruppe und die beiden anderen der genannten Reste für Wasserstoff stehen.

Das 3. erfindungsgemäße Verfahren zur Herstellung von erfindungsgemäßen Diisocyanaten bzw. Diisocyanatgemischen ist dadurch gekennzeichnet, daß man

a) ein Benzylhalogenid in Gegenwart von Friedel-Crafts-Katalysatoren oder Benzylalkohol in Gegenwart von Säure-Katalysatoren mit Alkylbenzolen zu einem im wesentlichen aus Kohlenwasserstoffen der Formel

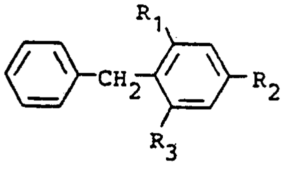

bestehenden Kondensat umsetzt,

b) das aus dem gemäß a) erhaltenen Kondensat destillativ in reiner Form erhaltene Alkyldiphenylmethan-Isomerengemisch der zuletzt gennanten Formel einer Dinitrierung unterzieht.

c) die gemäß b) erhaltenen Dinitroverbindungen durch Hydrierung bzw. Reduktion der Nitrogruppen in die entsprechenden Diamine überführt,

d) die gemäß c) erhaltenen Diamine durch Phosgenierung in die entsprechenden Diisocyanate überführt,

wobei man gegebenenfalls

e) aus den gemäß c) erhaltenen Diaminen vor der Phosgenierung und/oder aus den gemäß d) erhaltenen Diisocyanatgemischen das entsprechende im Gemisch mit bis zu 40 Gew.-% 2',3-Isomeren und mit bis zu 40 Gew.-%, bezogen auf Gesamtgemisch, an anderen Alkyl-substituierten Diisocyanatodiphenylmethan-Isomeren vorliegende 3,4'-Isomere in von Nebenprodukten befreiter Form isoliert.

Bei der Stufe a) des 3. erfindungsgemäßen Verfahrens wird anstelle eines Nitrobenzylhalogenids das unsubstituierte Benzylhalogenid oder Benzylalkohol eingesetzt.

Im Falle der Verwendung eines Benzylhalogenids gelten bezüglich der Durchführung der Stufe a) insbesondere bezüglich der Mengenverhältnisse der Reaktionspartner die bei der Schilderung des 1. und 2. erfindungsgemäßen Verfahrens gemachten Ausführungen. Vorzugsweise wird jedoch bei einem Molverhältnis von Alkyl-Benzol zu Benzylchlorid von 5 : 1 bis 20 : 1 gearbeitet, besonders bevorzugt ist 8 : 1 bis 15 : 1. Die Kondensationsreaktion kann jedoch im Extremfall in der Gasphase bei Temperaturen von bis zu 300 °C durchgeführt werden. Die bevorzugte Temperatur zur Durchführung der Stufe a) liegt jedoch innerhalb der für die Stufe a) des 2. erfindungsgemäßen Verfahrens gültigen Bereiche.

Im Falle der Verwendung von Benzylalkohol als Ausgangsmaterial werden als Katalysatoren schwerflüchtige starke Säuren eingesetzt wie z. B. Schwefelsäure, Phosphorsäure oder beispielsweise Sulfonsäuregruppen aufweisende Festbettkatalysatoren wie Sulfonsäuregruppen aufweisende Ionenaustauscher oder anorganische saure Zentren aufweisende Feststoffkatalysatoren (Tonsile, Zeolithe etc).

Bezüglich der Mengenverhältnisse der Reaktionspartner gelten die für die Reaktion zwischen Benzylchlorid und Alkylbenzol gemachten Ausführungen sinngemäß, d. h. auch hier wird das Alkylbenzol in einem diesen Ausführungen entsprechenden Überschuß eingesetzt. Die Reaktionstemperatur liegt hier im allgemeinen zwischen − 20 und 300, vorzugsweise 20 bis 110 °C. Das anfallende Kondensat wird beispielsweise durch Auswaschen mit Wasser bei homogener Katalyse, bzw. durch Filtrieren bei heterogener Katalyse vom Katalysator und durch Abdestillieren vom überschüssigen Alkylbenzol befreit und in destillativ von geringen Mengen an höhermolekularen Kondensaten befreiter Form der Stufe b) zugeführt.

Bei dieser Reaktionsstufe b) wird das Kondensat einer Dinitrierung unterzogen, wobei im Prinzip die bei der Schilderung der Stufe b) des 2. erfindungsgemäßen Verfahrens gemachten Ausführungen gelten, jedoch mit dem Unterschied, daß hier für jedes Mol an zu nitrierendem Kohlenwasserstoff eine solche Menge an Nitriersäure zur Verfügung steht, die ca. 2,0 bis 2,5 Mol Salpetersäure entspricht.

Die weitere Verarbeitung der so erhaltenen Alkyl-substituierten Dinitrodiphenylmethan-Isomeren erfolgt in völliger Analogie zu der bereits in Zusammenhang mit dem 1. oder 2. erfindungsgemäßen Verfahren gemachten Ausführungen. Auch hier kann, wie beim 1. erfindungsgemäßen Verfahren beschrieben, durch partielle Kristallisation auf der Nitro-Stufe das 4-Alkyl-3,4'-Isomere dargestellt werden.

Beim 3. erfindungsgemäßen Verfahren werden im Gemisch mit bis zu 40 Gew.-%, vorzugsweise im Gemisch mit 10 bis 25 Gew.-%, bezogen auf Gesamtgemisch an Diisocyanaten der Formel

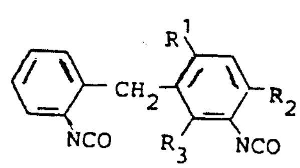

und mit bis zu 40 Gew.-%, bezogen auf Gesamtgemisch an anderen Alkyl-substituierten Diisocyanatodiphenylmethan-Isomeren vorliegende Diisocyanate der Formel

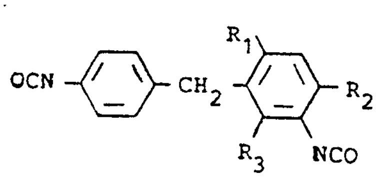

erhalten, wobei in diesen und auch der zuletzt genannten Formel für die durch die Kondensationsreaktion entstehenden Kohlenwasserstoffe jeweils einer der Reste $R_1$, $R_2$ oder $R_3$ für eine Alkylgruppe und die beiden anderen Reste jeweils für Wasserstoff stehen.

Im Falle der Herstellung von Alkyl-substituierten erfindungsgemäßen Diisocyanaten bzw. Diisocyanatgemischen entstehen, vom 1. erfindungsgemäßen Verfahren unter Verwendung 1-Alkyl-4-nitro-benzol abgesehen, stets Gemische von sich bezüglich der Stellung des Alkylsubstituenten unterscheidenden Isomeren, wobei meistens das erfindungsgemäße 3,4'-Diisocyanato-4-alkyl-diphenyl-methan den Hauptbestandteil bildet. Bezüglich der Eigenschaften der erfindungsgemäßen Diisocyanatgemische und insbesondere bezüglich deren Eignung als Ausgangsmaterial zur Herstellung von Polyurethanen ist die Stellung des Alkylsubstituenten bzw. der jeweilige Anteil der jeweiligen, sich durch die Stellung des Alkylsubstituenten unterscheidenden Isomeren von völlig untergeordneter Bedeutung. Die erfindungsgemäßen Polyisocyanatgemische stellen technische Gemische dar, die, wie dargelegt, oftmals untergeordnete Mengen, d. h. bis zu 40 Gew.-% an analytisch, d. h. insbesondere gaschromatographisch nicht eindeutig charakterisierbaren Bestandteilen enthalten. Es handelt sich hierbei im allgemeinen um einen Alkylsubstituenten aufweisende Diisocyanato-diphenylmethan-Isomere, deren NCO-Gruppen in 2,4', 4,4'- oder 3,3'-Stellung angeordnet sind, bzw. um Gemische derartiger Isomerer. Der Gehalt der erfindungsgemäßen Gemische an diesen Nebenprodukten in den angegebenen Grenzen beeinträchtigt jedoch in keiner Weise deren vorteilhaften Eigenschaften. Allen erfindungsgemäßen Diisocyanaten bzw. Diisocyanatgemischen gemeinsam sind die eingangs erwähnten Vorteile im Vergleich zu TDI bzw. MDI. Die erfindungsgemäßen Diisocyanate bzw. Diisocyanatgemische. Die Viskosität der erfindungsgemäßen Diisocyanate bzw. Diisocyanatgemische bei 25 °C liegt im allgemeinen im Bereich von 10 bis 50 mPa.s.

Die erfindungsgemäßen Diisocyanate bzw. Diisocyanatgemische stellen besonders wertvolle neuartige Ausgangsmaterialien zur Herstellung von Polyurethankunststoffen dar. Die neuen erfindungsgemäßen Diisocyanate bzw. Diisocyanatgemische können bei allen bislang bekanntgewordenen Verfahren zur Polyurethankunststoffherstellung unter Verwendung von TDI und/oder MDI an Stelle dieser Polyisocyanate des Standes der Technik eingesetzt werden.

## Beispiel 1

(1. erfindungsgemäßes Verfahren)

a) Bei Raumtemperatur wurden 171,5 g (1 Mol) 4-Nitrobenzylchlorid in 755 g (5 Mol) 1-Ethyl-2-nitrobenzol gelöst und mit 13,2 g (0,1 Mol) wasserfreiem Aluminiumtrichlorid versetzt.

Unter Rühren wurde das Reaktionsgemisch langsam auf 120 °C geheizt und bis zum Nachlassen der Chlorwasserstoffentwicklung bei dieser Temperatur gehalten. Nach insgesamt 8 Stunden konnte im Reaktionsgemisch kein Nitrobenzylchlorid mehr nachgewiesen werden.

Nach dem Abkühlen auf Raumtemperatur wurde mit 500 ml Methylenchlorid verdünnt, einmal mit 200 ml halbkonzentrierter Salzsäure extrahiert und dreimal mit je 500 ml Wasser gewaschen. Nach dem Trocknen wurde zunächst das Methylenchlorid und dann das überschüssige 1-Ethyl-2-nitro-benzol abgetrennt, gefolgt von einer Wasserdampfdestillation.

Der Rückstand (275 g) enthielt nach gaschromatographischer Analyse in seinem verdampften Anteil :

3,0 Gew.-% nicht-identifizierte Dinitrozweikernverbindungen ;
68,0 Gew.-% 4-Ethyl-3,4'-dinitrodiphenylmethan ;
29,0 Gew.-% 2-Ethyl-3,4'-dinitrodiphenylmethan.

b) 250 g (0,875 Mol) des Nitroproduktes aus Stufe a) wurden in 600 ml Methanol aufgenommen, mit 30 g frischem Raney-Nickel versetzt und bei 50 °C mit Wasserstoff bei 50 bar Druck bis zur Druckkonstanz reduziert. Nach Beendigung der exothermen Reaktion wurde noch eine Stunde bei 100 °C und 50 bar Wasserstoffdruck gehalten. Danach wurde entspannt, der Katalysator abfiltriert und Methanol und Wasser abdestilliert.

Der Rückstand (199 g) bestand nach gaschromatographischer Analyse aus :

0,7 % niedrigsiedende Produkte
91,5 % Diamine
7,8 % höhersiedende Produkte

c) Das Rohamin (199 g) wurde in 1 250 ml Monochlorbenzol gelöst und bei 0 °C unter Rühren und Kühlen zu einer Lösung von 400 g Phosgen in 1 250 ml Monochlorbenzol zugetropft. Anschließend wurde unter weiterem Durchleiten von Phosgen das Reaktionsgemisch langsam aufgeheizt und 1 Stunde am Rückfluß gekocht.

Im Wasserstrahlvakuum wurde anschließend das Monochlorbenzol abgezogen und bei weiter reduziertem Druck von 0,13 mbar (0,1 Torr) das Isocyanat destilliert. Nach einem geringen Vorlauf (ca. 20 ml) ging bei 158 bis 163 °C eine Diisocyanatfraktion (195 g, 0,7 Mol) über bei ca. 35 g Destillationsrückstand.

Die Diisocyanatfraktion enthielt nach GC neben einem nicht näher identifizierten Diisocyanat (ca. 3

Gew.-%) zu 97 % ein Gemisch aus 2- und 4-Ethyl-3,4'-diisocyanatodiphenylmethan (Gewichtsverhältnis ca. 30 : 70).

Beispiel 2

(1. erfindungsgemäßes Verfahren)

a) 250 g (1,45 Mol) 4-Nitrobenzylchlorid wurden zusammen mit 587 g (3,89 Mol) 1-Ethyl-4-nitrobenzol vermischt und nach dem Homogenisieren mit 16,2 g (0,1 Mol) wasserfreiem Eisen-III-chlorid versetzt.

Die Durchführung der Reaktion und die Aufarbeitung der Produkte erfolgte analog Beispiel 1a) und ergab einen festen Rückstand (357 g), der durch Umkristallisieren aus Essigester/Ethanol 1 : 1 gereinigt wurde. Es resultierte reines 6-Ethyl-3,4'-dinitrodiphenylmethan (Fp. 125 bis 126 °C).

b) Von der gereinigten Nitrostufe wurden 40 g (0,14 Mol) durch Reduktion mit Eisenpulver in das Amin übergeführt (31 g = 99 % der Theorie).

c) Das Amin von b) wurde analog Beispiel 1c) durch Phosgenieren in das Isocyanat übergeführt. Das Isocyanat (37 g) wurde bei reduziertem Druck destilliert. Die Hauptfraktion (33,5 g = 0,12 Mol) bestand aus :

6-Ethyl-3,4'-diisocyanatodiphenylmethan ($Kp_{0,13}$ 158 °C, farblose Flüssigkeit, NCO-Gehalt 30,2 Gew.-%, Gehalt an hydrolisierbarem Chlor $< 0{,}01$ %).

Beispiel 3

(2. erfindungsgemäßes Verfahren)

a) 171,5 g (1 Mol) 4-Nitrobenzylchlorid wurden in 263 g (2,5 Mol) trockenem Ethylbenzol gelöst und bei 40 °C unter Rühren langsam zu 3 g wasserfreiem Eisen-III-chlorid in 800 g (7,5 Mol) trockenem Ethylbenzol zugetropft.

Unter Rühren wurde langsam bis auf 80 °C aufgeheizt, und bei dieser Temperatur gehalten. Nach beendeter Chlorwasserstoffentwicklung wurde 2 Stunden bei 70 bis 80 °C nachgerührt und nach dem Abkühlen dreimal mit je 500 ml Wasser säurefrei gewaschen.

Nach dem Trocknen und Abdestillieren des überschüssigen Ethylbenzols wurde der Rückstand (238 g) bei vermindertem Druck von 0,13 mbar (0,1 Torr) destilliert. Zwischen 135 und 145 °C gingen 227 g (94,2 % der Theorie) eines niederviskosen hellgelben Öles über, es verblieben 6 g Rückstand.

In einem Parallelansatz mit einem Ethylbenzolüberschuß von 5 : 1 betrug die Ausbeute 219 g (91 % der Theorie) an destilliertem Produkt praktisch gleicher Zusammensetzung.

Das GC-Spektrum zeigte :

ca. 2 Gew.-% nicht-identifizierte Verbindung
55 Gew.-% 2- (und 3-) Ethyl-4'-nitrodiphenylmethan
45 Gew.-% 4-Ethyl-4'-nitrodiphenylmethan

b) 241,0 g (1 Mol) des destillierten Gemisches der 4-Nitroethyldiphenylmethane wurden in 400 ml Methylenchlorid bei 20 °C vorgelegt. Unter Rühren und Kühlen wurden bei dieser Temperatur Nitriersäure zugegeben, bestehend aus einer Mischung von

71 g (1,1 Mol) 98 %iger $HNO_3$ und
110 g (1,1 Mol) 98 %iger $H_2SO_4$.

Nach beendeter Zugabe wurde nach 60 Minuten bei 20 °C nachgerührt und dann die Mischsäure abgetrennt. Die verbleibende organische Phase wurde zweimal mit je 200 ml Wasser, einmal mit 2 %iger Natriumcarbonatlösung und noch zweimal mit Wasser gewaschen. Anschließend wurde das Methylenchlorid bei Normaldruck bis 90 °C Sumpftemperatur abdestilliert, die Lösungsmittelreste wurden mit Wasserdampf ausgetrieben. Das resultierende Produkt (274 g ≙ 96 % der Theorie) wurde im Wasserstrahlvakuum bei 90 °C/16 mbar (12 mm Hg) entwässert. Es enthielt nach GC an verdampfbaren Anteilen :

ca. 7,0 Gew.-% niedrigsiedende Bestandteile
0,2 Gew.-% mononitrierte Ethyldiphenylmethane
92,6 Gew.-% dinitrierte Ethyldiphenylmethane

c) 250 g des Rohproduktes aus Stufe b) wurden in 650 ml Methanol aufgenommen, mit 20 g frischem Raney-Nickel versetzt und bei 50 °C mit Wasserstoff bei 50 bar bis zur Druckkonstanz reduziert. Nach Beendigung der exothermen Reaktion wurde noch 1 Stunde bei 80 °C und 50 bar Wasserstoffdruck gehalten. Danach wurde entspannt, der Katalysator abfiltriert und Methanol und Wasser abdestilliert bis 85 °C/20 mbar (15 Torr). Der Rückstand von 190 g wurde bei einem Vakuum von 0,13 mbar (0,1 Torr)

destilliert. Nach einem Vorlauf von 8 g (50 bis 150 °C) wurden als Hauptfraktion zwischen 150 und 250 °C 167 g Destillat erhalten naben einem Rückstand von 11 g.

Gemäß Gaschromatogramm bestand die Hauptfraktion aus :

< 1 Gew.-% niedrigsiedende Verbindung
97-98 Gew.-% Diamine
< 2 Gew.-% höherkernige Verbindung.

d) 165 g (0,75 Mol) der Diaminfraktion von c) wurden analog Beispiel 1c) durch Phosgenierung in die Isocyanate überführt.

Im Wasserstrahlvakuum wurde anschließend das Monochlorbenzol abgezogen und bei weiter reduziertem Druck (ca. 0,13 mbar) (ca. 0,1 Torr) das Isocyanat destilliert. Nach einem Vorlauf von ca. 25 ml ging bei 150 bis 175 °C eine Diisocyanatfraktion (189 g ; 0,68 Mol) über. Der Destillationsrückstand betrug 12,5 g. Die Diisocyanatfraktion bestand nach GC aus :

ca. 75 Gew.-% 2-, 4- und 6-Ethyl-3,4'-diisocyanatodiphenylmethan und
ca. 25 Gew.-% anderen Isomeren.

Beispiel 4

(2. erfindungsgemäßes Verfahren)

a) 171,5 g (1 Mol) 4-Nitrobenzylchlorid wurden in 300 g (2,5 Mol) trockenem Isopropylbenzol gelöst und bei 40 °C unter Rühren langsam zu 5 g wasserfreiem Eisen-III-chlorid in 900 g (7,5 Mol) trockenem Isopropylbenzol zugetropft.

Das Reaktionsgemisch wurde langsam auf 90 °C erhitzt und gerührt, bis die HCl-Abspaltung nahezu beendet war. Danach wurde eine weitere Stunde bei 90 °C nachgerührt. Die Aufarbeitung erfolgte analog Beispiel 3a). Bei der Destillation des nach Abziehen des Isopropylbenzols verbleibenden Rückstandes gingen bei 0,13 mbar (0,1 Torr) zwischen 150 und 160 °C 238 g (0,93 Mol) einer Fraktion über, die nach GC zu 99 Gew.-% aus Isopropyl-4-nitrodiphenylmethan bestand. Der Destillationsrückstand betrug 6 g.

Nach GC war die Zusammensetzung :

54 Gew.-% 2- (und 3-) Isopropyl-4'-nitrodiphenylmethan
46 Gew.-% 4-Isopropyl-4'-nitrodiphenylmethan.

In einem Parallelansatz mit einem Isopropylüberschuß von 5 : 1 betrug die Ausbeute 252 g (0,9 Mol) an destilliertem Produkt praktisch gleicher Zusammensetzung.

b) 255 g (1 Mol) Isopropyl-4-nitro-diphenylmethan aus Stufe a) wurden einer Zweitnitrierung unterworfen analog Beispiel 3b) und aufgearbeitet. Das Rohprodukt 282 g enthielt nach GC in seinem verdampfbaren Anteil :

9,2 Gew.-% niedrigsiedende Anteile
11,0 Gew.-% mononitrierte Isopropyldiphenylmethane
79,8 Gew.-% dinitrierte Isopropyldiphenylmethane.

c) 250 g Rohprodukt der Stufe b) wurden analog Beispiel 3c) durch Reduktion in die Aminstufe übergeführt (Ausbeute : 196 g). Der Rückstand wurde fraktioniert destilliert (Kp 165 bis 195 °C/0,13 mbar (0,1 Torr).

d) Aus der Hauptfraktion der Amine wurde 140 g (0,5 Mol) analog Beispiel 1d) durch Phosgenieren in die Isocyanate übergeführt.

Im Wasserstrahlvakuum wurde anschließend das Monochlorbenzol abgezogen und bei weiter reduziertem Druck von 0,13 mbar (0,1 Torr) das Isocyanat destilliert.

Nach einem Vorlauf von ca. 15 ml, der auch noch Chlorbenzol enthielt, ging bei 160 bis 165 °C eine Diisocyanatfraktion 131 g (0,48 Mol) über. Der Destillationsrückstand betrug 14 g.

Die Diisocyanatfraktion hatte folgende Zusammensetzung nach GC :

ca. 65 Gew.-% Isopropyl-3,4'-diisocyanatodiphenylmethane
ca. 35 Gew.-% andere Isomere,

wobei die Isopropylreste der Hauptkomponente in 2-, 4- und 6-Stellung angeordnet sind.

Beispiel 5

(3. erfindungsgemäßes Verfahren)

a) Unter Stickstoff wurden 2,12 kg (20 Mol) trockenes Ethylbenzol vorgelegt und mit 10 g

wasserfreiem Eisen-III-chlorid versetzt. Unter Rühren und bei 60 °C wurden anschließend 253 g (2 Mol) trockenes Benzylchlorid zugetropft, wobei gasförmiger Chlorwasserstoff entwich.

Nach beendeter Zugabe wurde noch 60 Minuten nachgerührt, abgekühlt und dreimal mit je 500 ml Wasser säurefrei gewaschen. Anschließend wurde aus der organischen Phase durch Destillation das überschüssige Ethylbenzol abgetrennt.

Das verbleibende Kohlenwasserstoffgemisch (ca. 350 g) bestand aus

82 Gew.-% Zweikernisomeren
12-15 Gew.-% Dreikernisomeren
< 5 Gew.-% höherkernigen Bestandteilen.

Durch Destillation bei vermindertem Druck wurde zunächst die Zweikernfraktion (153 bis 156 °C/20 mbar (15 Torr)) und anschließend die Dreikernfraktion (150 bis 175 °C/0,13 mbar (0,1 Torr)) abgetrennt.

Die Zweikernfraktion enthielt nach GC- und NMR-Analyse

ca. 40 Gew.-% 2-Ethyldiphenylmethan und
ca. 60 Gew.-% 4-Ethyldiphenylmethan.

b) 294 g (1,5 Mol) des nach a) erhaltenen Gemisches der Zweikernisomeren des Ethyldiphenylmethans wurden in 900 ml Methylenchlorid bei 0 bis 10 °C vorgelegt. Unter Rühren und Kühlen wurde bei dieser Temperatur Nitriersäure zugegeben, bestehend aus einer Mischung von

209 g (3,25 Mol) 98 %iger $HNO_3$ und
325 g (3,25 Mol) 98 %iger $H_2SO_4$.

Bei 20 °C wurde nach beendeter Zugabe noch 2 Stunden nachgerührt und dann die Mischsäure abgetrennt. Die organische Phase wurde zweimal mit je 400 ml Wasser, einmal mit 2 %iger Kaliumcarbonatlösung und noch zweimal mit Wasser gewaschen. Anschließend wurde das Methylenchlorid bei Normaldruck bis 90 °C Sumpftemperatur abdestilliert und die Lösungsmittelreste mit Wasserdampf ausgetrieben. Im Wasserstrahlvakuum wurde bis 100 °C entwässert. Der Rückstand (401 g = 93,5 % der Theorie, Fp. 40 °C) enthielt in seinem verdampfbaren Anteil

8,3 Gew.-% niedrigsiedende Bestandteile
1 Gew.-% mononitrierte Ethyldiphenylmethane
91,3 Gew.-% dinitrierte Ethyldiphenylmethane.

Die Dinitrofraktion enthielt ca. 15 bis 20 Gew.-% an 2',3-Dinitroverbindungen ; 55 bis 60 Gew.-% an 3,4'-Dinitroverbindungen und 30 Gew.-% an anderen Isomeren.

c) 400 g des Rohgemisches der Nitrierstufe wurde in 1 000 ml Methanol aufgenommen, mit 40 g frischem Raney-Nickel versetzt und bei 50 °C mit einem Wasserstoffdruck von 50 bar reduziert. Nach Beendigung der exothermen Reaktion wurde bei 100 °C noch 1 Stunde lang der Wasserstoffdruck auf 100 bar gehalten. Danach wurde entspannt, der Katalysator abfiltriert und Methanol und Wasser wurden abdestilliert.

Der Rückstand wurde bei reduziertem Druck destilliert, wobei zunächst ein Vorlauf von ca. 50 g niedrigsiedender Verbindungen mit Wasserresten überging.

Bei 160 bis 200 °C (0,13 mbar) (0,1 Torr) destillierte die Hauptmenge des Amingemisches (258 g = 1,14 Mol). Der Rückstand im Destillationskolben ergab 15 g.

d) 250 g (1,1 Mol) des Gemisches der Diaminoethyldiphenylmethane (enthält noch < 1 % Monoamin) wurden in 1,25 l Monochlorbenzol gelöst und bei 0 °C unter Rühren und Kühlen zu einer Lösung von 400 g Phosgen in 1,25 l Monochlorbenzol langsam zugetropft. Anschließend wurde unter weiterem Durchleiten von Phosgen das Reaktionsgemisch langsam aufgeheizt bis das Monochlorbenzol am Rückfluß kochte und so eine Stunde gehalten.

Nach dem Abziehen des Monochlorbenzols im Wasserstrahlvakuum wurde bei einem reduzierten Druck von 0,13 mbar (0,1 Torr) das Isocyanat destilliert. Nach einem Vorlauf von ca. 10 g erhielt man bei 155 bis 175 °C das Isomerengemisch der Isocyanate (291 g, NCO-Gehalt 30,2 g) als hellgelbes Öl, das selbst bei 0 °C noch flüssig blieb. Der Destillationsrückstand ergab 10 g.

Die Isomerenverteilung der Diisocyanatfraktion entspricht der Isomerenverteilung der Dinitrofraktion.

**Ansprüche**

1. Gegebenenfalls im Gemisch mit bis zu 40 Gew.-%, bezogen auf Gesamtgemisch, an Diisocyanaten der Formel

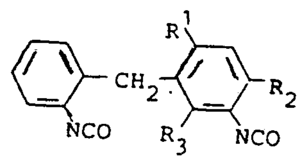

und gegebenenfalls mit bis zu 40 Gew.-%, bezogen auf Gesamtgemisch, an anderen, $C_2$-$C_{12}$-Alkyl-substituierten Diisocyanatodiphenylmethan-Isomeren vorliegende Diisocyanate der Formel

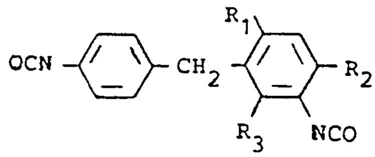

wobei in diesen Formeln die Reste $R_1$, $R_2$ und $R_3$ jeweils gleich oder verschieden sind und Wasserstoff oder eine $C_2$-$C_{12}$-Alkylgruppe bedeuten, mit der Einschränkung, daß in jeder der beiden Formeln jeweils zwei der Reste $R_1$, $R_2$ und $R_3$ für Wasserstoff stehen und wobei einer der Reste $R_1$, $R_2$ oder $R_3$ für eine Alkylgruppe mit 2 bis 12 Kohlenstoffatomen steht.

2. Diisocyanate bzw. Diisocyanatgemische gemäß Anspruch 1, dadurch gekennzeichnet, daß in den beiden der in Anspruch 1 genannten Formeln jeweils einer der Reste $R_1$, $R_2$ oder $R_3$ für eine Ethylgruppe steht.

3. Diisocyanat gemäß Anspruch 1 der Formel

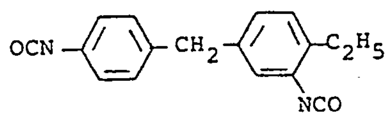

4. Diisocyanat gemäß Anspruch 1 der Formel

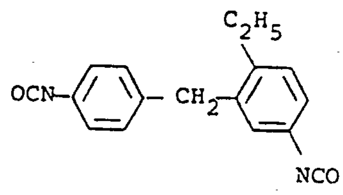

5. Verfahren zur Herstellung von Diisocyanaten der Formel

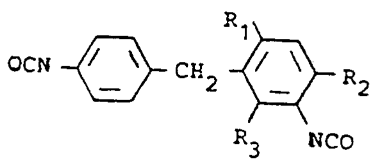

in welcher $R_1$, $R_2$ und $R_3$ die in Anspruch 1 genannte Bedeutung haben, dadurch gekennzeichnet, daß man

a) ein 4-Nitrobenzylhalogenid in Gegenwart von Friedel-Crafts-Katalysatoren mit 1-Alkyl-2-nitrobenzol und/oder 1-Alkyl-4-nitro-benzol und/oder technischen Gemischen dieser Isomeren mit bis zu 15 Gew.-%, bezogen auf Gesamtgemisch, an 1-Alkyl-3-nitro-benzol zu Dinitroverbindungen der Formel

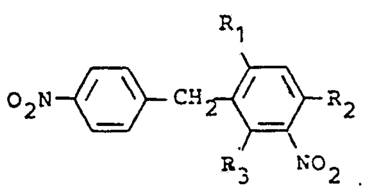

gegebenenfalls im Gemisch mit bis zu 20 Gew.-% an anderen isomeren Dinitro-diphenylmethanen umsetzt, das Umsetzungsprodukt von eingesetztem Katalysator befreit,

b) das gemäß a) erhaltene Umsetzungsprodukt durch Hydrierung oder Reduktion der Nitrogruppen in die entsprechenden aromatischen Diaminoverbindungen überführt und

c) die gemäß b) erhaltenen Diaminoverbindungen durch Phosgenierung in die Diisocyanate überführt,

wobei man gegebenenfalls

d) aus den gemäß b) erhaltenen Diaminen vor der Phosgenierungsreaktion und/oder aus den gemäß c) erhaltenen Diisocyanaten die entsprechenden 3,4'-Diamino- bzw. 3,4'-Diisocyanato-Isomeren destillativ in von Nebenprodukten befreiter Form isoliert.

6. Verfahren zur Herstellung von Diisocyanaten der Formel

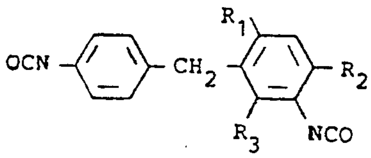

in welcher $R_1$, $R_2$ und $R_3$ die in Anspruch 1 genannte Bedeutung haben, dadurch gekennzeichnet, daß man

a) ein 4-Nitrobenzylhalogenid in Gegenwart von Friedel-Crafts-Katalysatoren mit Alkylbenzolen zu einem Gemisch von Mononitroverbindungen der Formel

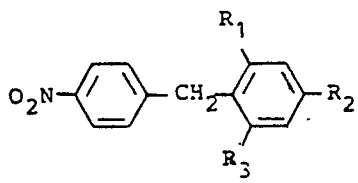

umsetzt, das Umsetzungsprodukt vom Katalysator befreit,

b) das gemäß a) erhaltene Umsetzungsprodukt einer Nitrierungsreaktion unter Bildung von Dinitroverbindungen der Formel

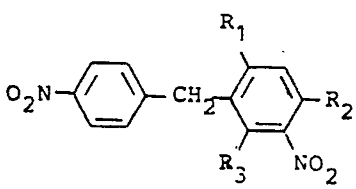

unterzieht,

c) die gemäß b) erhaltenen Dinitroverbindungen durch Hydrierung bzw. Reduktion der Aminogruppen in die entsprechenden aromatischen Diaminoverbindungen überführt und

d) die gemäß c) erhaltenen Diaminoverbindungen durch Phosgenierung in die entsprechenden Diisocyanate überführt,

wobei man gegebenenfalls

e) aus den gemäß c) erhaltenen Diaminen vor der Phosgenierungsreaktion und/oder aus den gemäß d) erhaltenen Diisocyanaten die entsprechenden 3,4'-Diamino- bzw. 3,4'-Diisocyanato-Isomeren destillativ in von Nebenprodukten befreiter Form isoliert.

7. Verfahren zur Herstellung von im Gemisch mit bis zu 40 Gew.-%, bezogen auf Gesamtgemisch, an Diisocyanaten der Formel

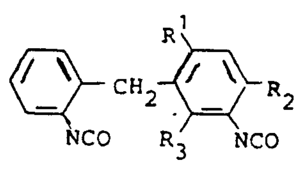

und mit bis zu 40 Gew.-%, bezogen auf Gesamtgemisch, an anderen $C_2$-$C_{12}$-Alkyl-substituierten Diisocyanatodiphenylmethan-Isomeren vorliegende Diisocyanate der Formel

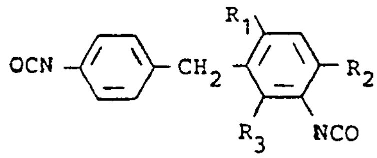

wobei in beiden Formeln die Reste $R_1$, $R_2$ und $R_3$ die in Anspruch 1 genannte Bedeutung haben, dadurch gekennzeichnet, daß man

a) ein Benzylhalogenid in Gegenwart von Friedel-Crafts-Katalysatoren oder Benzylalkoholen in Gegenwart von Säure-Katalysatoren mit Alkylbenzolen zu einem im wesentlichen aus Kohlenwasserstoffen der Formel

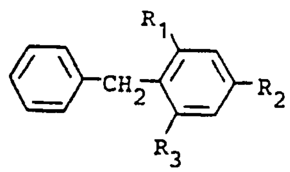

bestehenden Kondensat umsetzt,

b) das aus dem gemäß a) erhaltenen Kondensat destillativ in reiner Form erhaltene Alkyldiphenylmethan-Isomerengemisch der zuletztgenannten Formel einer Dinitrierung unterzieht,

c) die gemäß b) erhaltenen Dinitroverbindungen durch Hydrierung bzw. Reduktion der Nitrogruppen in die entsprechenden Diamine überführt,

d) die gemäß c) erhaltenen Diamine durch Phosgenierung in die entsprechenden Diisocyanate überführt,

wobei man gegebenenfalls

e) aus den gemäß c) erhaltenen Diaminen vor der Phosgenierung und/oder aus den gemäß d) erhaltenen Diisocyanatgemischen das entsprechende im Gemisch mit bis zu 40 Gew.-% 2,3'-Isomeren und mit bis zu 30 Gew.-% bezogen auf Gesamtgemisch, an anderen Alkyl-substituierten Diisocyanatodiphenylmethan-Isomeren vorliegende 3,4'-Isomere in von Nebenprodukten befreiter Form isoliert.

8. Verwendung der Diisocyanate bzw. Diisocyanatgemische gemäß Ansprüchen 1 bis 4 als Isocyanatkomponente bei der Herstellung von Polyurethankunststoffen nach dem Isocyanat-Polyadditionsverfahren.

**Claims**

1. Diisocyanates corresponding to the following formula

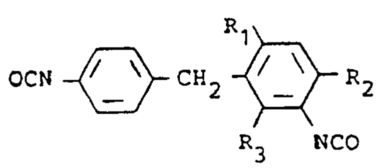

optionally present in admixture with up to 40 % by weight, based on the mixture as a whole, of diisocyanates corresponding to the following formula

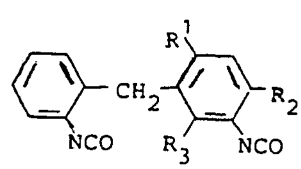

and optionally with up to 40 % by weight, based on the mixture as a whole, of other, $C_2$-$C_{12}$-alkyl-substituted diisocyanato-diphenyl methane isomers, the radicals $R_1$, $R_2$ and $R_3$ in the above formulae being the same or different and representing hydrogen or a $C_2$-$C_{12}$-alkyl group, with the proviso that, in each of the two general formulae, two of the radicals $R_1$, $R_2$ and $R_3$ represent hydrogen and one of the radicals $R_1$, $R_2$ or $R_3$ represents an alkyl group containing from 2 to 12 carbon atoms.

2. Diisocyanates or diisocyanate mixtures as claimed in Claim 1, characterised in that, in the two formulae given in Claim 1, one of the radicals $R_1$, $R_2$ or $R_3$ represents an ethyl group.

3. A diisocyanate as claimed in Claim 1 corresponding to the following formula

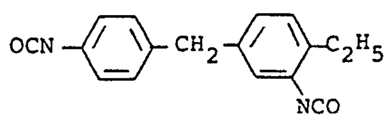

4. A diisocyanate as claimed in Claim 1, corresponding to the following formula

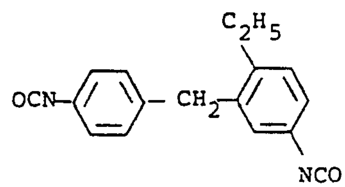

5. A process for producing diisocyanates corresponding to the following formula

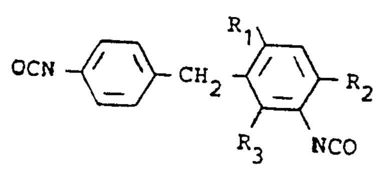

in which $R_1$, $R_2$ and $R_3$ are as defined in Claim 1, characterised in that

a) a 4-nitrobenzyl halide is reacted in the presence of Friedel-Crafts catalysts with 1-alkyl-2-nitrobenzene and/or 1-alkyl-4-nitrobenzene and/or technical mixtures of these isomers with up to 15 % by weight, based on the mixture as a whole, of 1-alkyl-3-nitrobenzene to form dinitro compounds corresponding to the following formula

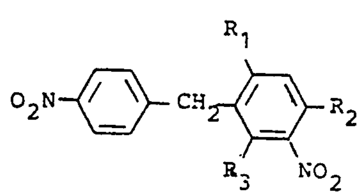

optionally in admixture with up to 20 % by weight of other isomeric dinitro-diphenyl methanes, after which the reaction product is freed from the catalyst used,

b) the reaction product obtained in accordance with a) is converted by hydrogenation or reduction of the nitro groups into the corresponding aromatic diamino compounds and

c) the diamino compounds obtained in accordance with b) are converted by phosgenation into the diisocyanates,

d) the corresponding 3,4'-diamino or 3,4'-diisocyanato-isomers optionally being isolated free from secondary products by distillation from the diamines obtained in accordance with b) before the phosgenation reaction and/or from the diisocyanates obtained in accordance with c).

6. A process for producing diisocyanates corresponding to the following formula

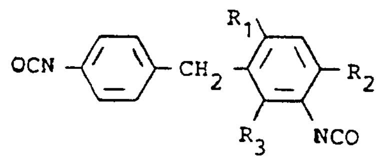

in which $R_1$, $R_2$ and $R_3$ are as defined in Claim 1, characterised in that

a) a 4-nitrobenzyl halide is reacted with alkyl benzenes in the presence of Friedel-Crafts catalysts to form a mixture of mononitro compounds corresponding to the following formula

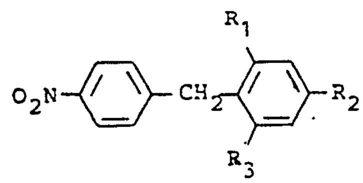

after which the reaction product is freed from catalyst,

b) the reaction product obtained in accordance with a) is subjected to a nitration reaction to form dinitro compounds corresponding to the following formula

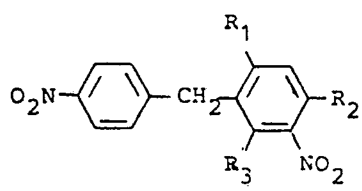

c) the dinitro compounds obtained in accordance with b) are converted by hydrogenation or reduction of the amino groups into the corresponding aromatic diamino compounds and

d) the diamino compounds obtained in accordance with c) are converted by phosgenation into the corresponding diisocyanates,

e) the corresponding 3,4'-diamino or 3,4'-diisocyanato-isomers optionally being isolated free from secondary products by distillation from the diamines obtained in accordance with c) before the phosgenation reaction and/or from the diisocyanates obtained in accordance with d).

7. A process for producing diisocyanates corresponding to the following formula

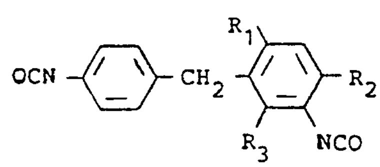

present in admixture with up to 40 % by weight, based on the mixture as a whole, of diisocyanates corresponding to the following formula

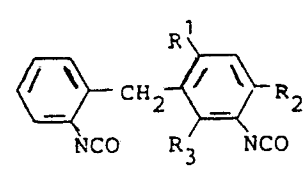

and with up to 40 % by weight, based on the mixture as a whole, of other $C_2$-$C_{12}$-alkyl-substituted diisocyanatodiphenyl methane isomers, the radicals $R_1$, $R_2$ and $R_3$ in both formulae being as defined in Claim 1, characterised in that

a) a benzyl halide or benzyl alcohol is reacted in the presence of Friedel-Crafts catalysts or acid

catalysts with alkyl benzenes to form a condensate essentially consisting of hydrocarbons corresponding to the following formula

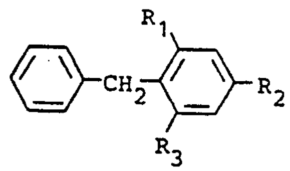

b) the alkyl diphenyl methane isomer mixture corresponding to the last formula which is obtained in pure form by distillation from the condensate obtained in accordance with a) is subjected to dinitration,

c) the dinitro compounds obtained in accordance with b) are converted by hydrogenation or reduction of the nitro groups into the corresponding diamines,

d) the diamines obtained in accordance with c) are converted by phosgenation into the corresponding diisocyanates,

e) the corresponding 3,4'-isomer present in admixture with up to 40 % by weight of 2,3'-isomers and with up to 30 % by weight, based on the mixture as a whole, of other alkyl-substituted diisocyanatodiphenyl methane isomers optionally being isolated free from secondary products from the diamines obtained in accordance with c) before phosgenation and/or from the diisocyanate mixtures obtained in accordance with d).

8. The use of the diisocyanates or diisocyanate mixtures claimed in Claims 1 to 4 as isocyanate component in the production of polyurethane plastics by the isocyanate polyaddition process.

**Revendications**

1. Diisocyanates de formule

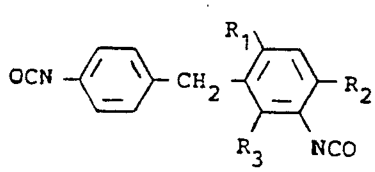

éventuellement présents en mélange avec jusqu'à 40 % en poids, calculé sur le mélange total, de diisocyanates de formule

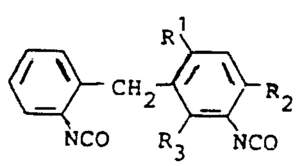

et éventuellement avec jusqu'à 40 % en poids, calculé sur le mélange total, d'autres isomères de diisocyanatodiphénylméthane substitués par un groupe alkyle en $C_2$-$C_{12}$,
les radicaux $R_1$, $R_2$ et $R_3$ de ces formules étant chacun identiques ou différents et représentant chacun un atome d'hydrogène ou un groupe alkyle en $C_2$-$C_{12}$, avec cette restriction que, dans chacune des deux formules, deux des radicaux $R_1$, $R_2$ et $R_3$ représentent chaque fois un atome d'hydrogène et un des radicaux $R_1$, $R_2$ ou $R_3$ représente un groupe alkyle contenant 2 à 12 atomes de carbone.

2. Diisocyanates ou mélanges de diisocyanates suivant la revendication 1, caractérisés en ce que, dans les deux formules mentionnées dans la revendication 1, un des radicaux $R_1$, $R_2$ ou $R_3$ représente chaque fois un groupe éthyle.

3. Diisocyanate suivant la revendication 1, caractérisé en ce qu'il répond à la formule

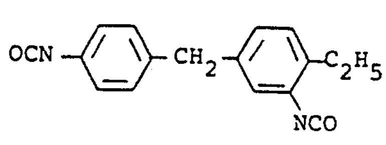

4. Diisocyanate suivant la revendication 1, caractérisé en ce qu'il répond à la formule

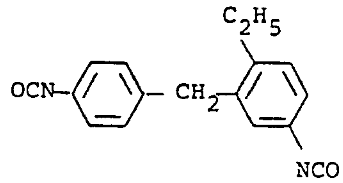

5. Procédé de préparation de diisocyanates de fòrmule

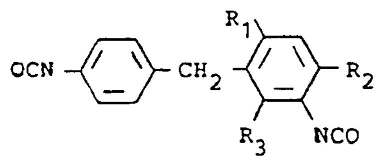

dans laquelle $R_1$, $R_2$ et $R_3$ ont les significations indiquées dans la revendication 1, caractérisé en ce que :

a) on fait réagir un halogénure de 4-nitrobenzyle, en présence de catalyseurs de Friedel-Crafts, avec un 1-alkyl-2-nitro-benzène et/ou un 1-alkyl-4-nitro-benzène et/ou avec des mélanges techniques de ces isomères avec jusqu'à 15 % en poids, calculé sur le mélange total, de 1-alkyl-3-nitro-benzène pour obtenir des dinitro-composés de formule

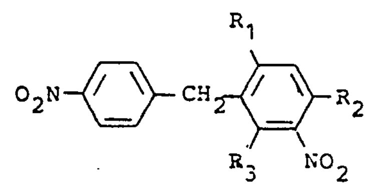

éventuellement en mélange avec jusqu'à 20 % en poids d'autres dinitro-diphénylméthanes isomères et on libère le produit réactionnel du catalyseur utilisé,

b) par hydrogénation ou réduction des groupes nitro, on transforme le produit réactionnel obtenu suivant l'étape a) en diamino-composés aromatiques correspondants, et

c) on transforme les diamino-composés obtenus suivant l'étape b) en diisocyanates par phosgénation

et éventuellement

d) à partir des diamines obtenues suivant l'étape b), avant la réaction de phosgénation et/ou à partir des diisocyanates obtenus suivant l'étape c), on isole, par distillation, les isomères 3,4'-diamino- ou 3,4'-diisocyanato correspondants sous une forme libérée des sous-produits.

6. Procédé de préparation de diisocyanates de formule

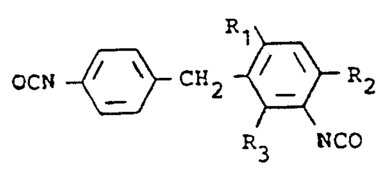

dans laquelle $R_1$, $R_2$ et $R_3$ ont les significations indiquées dans la revendication 1, caractérisé en ce que :

a) on fait réagir un halogénure de 4-nitrobenzyle, en présence de catalyseurs de Friedel-Crafts, avec des alkyl-benzènes pour obtenir un mélange de mononitro-composés de formule

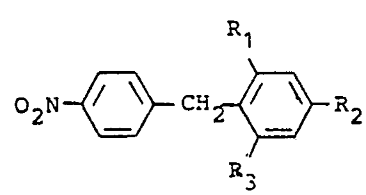

puis on libère le produit réactionnel du catalyseur,

b) on soumet le produit réactionnel obtenu suivant l'étape a) à une réaction de nitration avec formation de dinitro-composés de formule

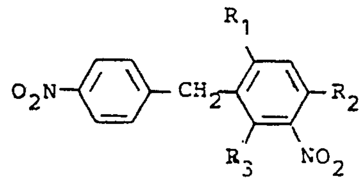

c) on transforme les dinitro-composés obtenus suivant l'étape b) en diamino-composés aromatiques correspondants par hydrogénation ou réduction des groupes amino, et

d) on transforme les diamino-composés obtenus suivant l'étape c) en diisocyanates correspondants par phosgénation,

et éventuellement

e) des diamines obtenues suivant l'étape c) avant la réaction de phosgénation et/ou des diisocyanates obtenus suivant l'étape d), on isole, par distillation, les isomères 3,4'-diamino ou 3,4'-diisocyanato correspondants sous forme libérée des sous-produits.

7. Procédé de préparation de diisocyanates de formule

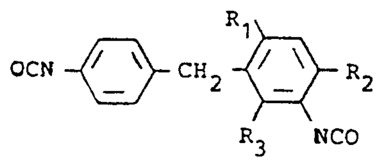

présents en mélange avec jusqu'à 40 % en poids, calculé sur le mélange total, de diisocyanates de formule :

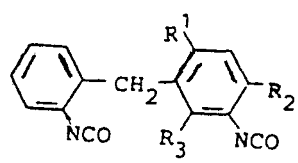

et avec jusqu'à 40 % en poids, calculé sur le mélange total, d'autres isomères de diisocyanato-diphénylméthane substitués par un groupe alkyle en $C_2$-$C_{12}$, les radicaux $R_1$, $R_2$ et $R_3$ ayant, dans les deux formules, la signification mentionnée dans la revendication 1, caractérisé en ce que :

a) on fait réagir un halogénure de benzyle, en présence de catalyseurs de Friedel-Crafts ou d'alcool benzylique, ainsi qu'en présence de catalyseurs acides, avec des alkylbenzènes pour obtenir un condensat constitué essentiellement d'hydrocarbures de formule :

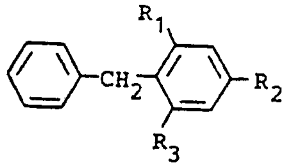

b) on soumet le mélange d'isomères d'alkyldiphénylméthanes de la formule mentionnée en dernier lieu et obtenu sous une forme pure par distillation à partir du condensat obtenu suivant l'étape a), à une dinitration ;

c) par hydrogénation ou réduction des groupes nitro, on transforme les dinitro-composés obtenus suivant l'étape b) en diamines correspondantes,

d) on transforme les diamines obtenues suivant l'étape c) en diisocyanates correspondants par phosgénation,

et éventuellement

e) des diamines obtenues suivant l'étape c) avant la phosgénation et/ou des mélanges de

diisocyanates obtenus suivant l'étape d), on isole, sous une forme libérée des sous-produits, les isomères 3,4' correspondants et présents en mélange avec jusqu'à 40 % en poids d'isomères 2',3 et avec jusqu'à 30% en poids, calculé sur le mélange total, d'autres isomères de diisocyanato-diphénylméthane substitués par un groupe alkyle.

8. Utilisation des diisocyanates ou des mélanges de diisocyanates suivant les revendications 1 à 4 comme composant isocyanate lors de la fabrication de matières synthétiques de polyuréthanes suivant le procédé de polyaddition d'isocyanates.